# EUROPÄISCHE PATENTSCHRIFT

(11) **EP 2 185 207 B1**
(45) Veröffentlichungstag und Bekanntmachung des Hinweises auf die Patenterteilung: **27.07.2011**
(21) Anmeldenummer: 08785289.3
(22) Anmeldetag: 01.08.2008
(51) Int. Cl.: A61L 24/04

(54) **KOMBINATION ZUM VERKLEBEN VON BIOLOGISCHEN GEWEBEN**
COMBINATION FOR AN ADHESIVE BONDING OF BIOLOGICAL TISSUES
COMBINAISON DE COLLAGE DE TISSUS BIOLOGIQUES

(30) Priorität: 03.08.2007 DE 102007038125
(43) Veröffentlichungstag der Anmeldung: 19.05.2010
(73) Patentinhaber: Aesculap AG, 78532 Tuttlingen/Donau (DE); Universität Rostock, 18051 Rostock (DE)
(72) Erfinder: ODERMATT, Erich, CH-8200 Schaffhausen (CH); WEGMANN, Jürgen, 78333 Stockach (DE); STERNBERG, Katrin, 18055 Rostock (DE); BEHREND, Detlef, 18119 Warnemünde (DE)
(74) Vertreter: Patentanwälte Ruff, Wilhelm, Beier, Dauster & Partner
(86) Internationale Anmeldenummer: PCT/EP2008/006348
(87) Internationale Veröffentlichungsnummer: WO 2009/018967

(56) Entgegenhaltungen:
- EP-A- 1 498 128
- WO-A-96/03159
- WO-A-98/19718
- WO-A-03/035122
- WO-A-2007/090373

## Beschreibung

Die Erfindung betrifft eine Kombination, insbesondere in Form einer medizinischen Klebstoffzusammensetzung, die zum Verkleben und/oder Fixieren von biologischen und/oder synthetischen Geweben geeignet ist.

Die modernen Wundverschlusstechniken basieren zunehmend auf dem Einsatz von sogenannten Gewebeklebern. Diese ermöglichen gewöhnlich eine einfache Handhabung. Weitere Vorteile ergeben sich aus einer verringerten Narbenbildung während der Wundheilung, einer geringeren Hypertrophie sowie dem Wegfall von postoperativen Folgebehandlungen.

Derzeit sind verschiedene Arten von Gewebeklebern kommerziell erhältlich. Die sogenannten Fibrinkleber setzen sich hauptsächlich aus Plasmaproteinen, insbesondere Fibrinogen und Thrombin, zusammen. Diese Kleber greifen aktiv in die Gerinnungskaskade ein und verfügen in der Regel über gute hämostatische Eigenschaften. Fibrinkleber werden jedoch normalerweise aus humanen oder tierischen Quellen hergestellt, wodurch ihre Bereitstellung gewöhnlich zeit- und vor allem kostenintensiv ist. Ein weiterer Nachteil besteht in einem nicht völlig auszuschließenden Infektionsrisiko für den Patienten.

Demgegenüber besitzen teilsynthetische Kleber, beispielsweise Gelatine-Resorcinol-Formaldehyd, zwar häufig eine höhere Haftkraft als die vorstehend beschriebenen Fibrinkleber. Doch verfügen sie im Vergleich zu den natürlichen Gewebeklebern in der Regel über schlechtere hämostatische Eigenschaften. Durch den natürlichen Bestandteil eines teilsynthetischen Klebers können grundsätzlich immer noch Infektionen übertragen werden. Zusätzlich sind teilsynthetische Kleber häufig mit einer toxischen Verbindung, beispielsweise Form- oder Glutaraldehyd, vernetzt.

Deswegen kommen mittlerweile zunehmend auch ausschließlich synthetisch hergestellte Gewebekleber zum Einsatz. Bei diesen Klebern handelt es sich hauptsächlich um Zusammensetzungen auf der Basis von Cyanoacrylaten. Derzeit werden unterschiedliche Cyanoacrylate als chirurgische Kleber eingesetzt. Beispielsweise setzt sich der Kleber Dermabond^{®} aus n-Octyl-2-Cyanoacrylat zusammen. Ein weiteres Beispiel ist das Kleberprodukt Histoacryl^{®}, welches n-Butyl-2-Cyanoacrylat enthält. Gewebekleber auf der Basis von Cyanoacrylaten sind gewöhnlich schnell aushärtbar. Die Verwendung von Cyanoacrylat-Klebern liefert zwar für die oberflächliche Wundversorgung befriedigende Ergebnisse. Doch bestehen gegen die Verwendung von Cyanoacrylat-Kleber zur Versorgung von inneren Wunden aus toxikologischer Sicht gewissen Bedenken. Hinzu kommt, dass Cyanoacrylat-Verbindungen mit biologischen Geweben unter stark exothermer Reaktion reagieren, wodurch gesunde Zellen im Wundbereich nachhaltig beschädigt werden können.

Da sowohl teil- als auch vollsynthetische Gewebekleber bei ihren medizinischen Verwendungen gewissen Limitierungen unterworfen sind, wird ständig nach alternativen Klebstoffzusammensetzungen gesucht. Eine alternative Klebstoffzusammensetzung ist beispielsweise in der EP 1. 719 530 A2 beschrieben. Die dort offenbarten Zusammensetzungen weisen jedoch eine langsame Aushärtung auf, wodurch ihre medizinische Anwendbarkeit ebenfalls eingeschränkt ist.

Die WO 96/03159 A1 offenbart eine Klebstoffzusammensetzung zum Verkleben von Geweben auf Basis eines Proteins und eines Oligolactoneinheiten aufweisenden Vernetzungsmittels.

Gegenstand der WO 03/035122 A1 ist eine Zusammensetzung zum Verkleben von biologischem Gewebe auf Basis eines Aminogruppen tragenden Polymers und eines mindestens drei Aldehydgruppen aufweisenden Aldehyds, wobei die Zusammensetzung frei von Eiweiß ist.

Aus der EP 1498128 A1 geht eine medizinische Zusammensetzung auf Basis eines photovernetzbaren Chitosanderivats und Promotors für die Wundheilung hervor.

Die WO 98/19718 A1 bezieht sich auf ein medizinisches Produkt, welches ein mit Genipin vernetztes und Aminogruppen enthaltendes Biopolymer aufweist.

Die nachveröffentlichte WO 2007/090373 A2 betrifft eine Kombination, welche ein Peptid sowie ein endständig funktionalisiertes Oligolacton umfasst.

Aus der US 4,740,534 geht ein chirurgischer Kleber auf der Basis eines Urethanpräpolymers und einer ungesättigten Cyanoverbindung hervor. Bei der Cyanoverbindung kann es sich um Cyanoacrylate oder Cyanoacrylnitrile handeln. Wie bereits erwähnt, ist insbesondere der Einsatz von Cyanoacrylat-Verbindungen mit gewissen Nachteilen behaftet.

Ein weiterer biologischer Kleber ist aus der WO 2005/118011 A1 bekannt. Die dort beschriebene Klebstoffzusammensetzung weist eine Mischung aus vernetzungsfähigen Präpolymeren auf, die mit Isocyanatgruppen modifiziert sind. Nachteilig bei dieser Art von Klebstoffzusammensetzung ist ihre vergleichsweise geringe Viskosität, wodurch insbesondere bei stark blutenden Wunden grundsätzlich die Gefahr besteht, dass die Zusammensetzung aus dem Wundbereich weggespült wird. Auch diese Klebstoffzusammensetzung weist eine vergleichsweise lange Gelierungs- oder Aushärtezeit auf. Dies kann insbesondere bei starken Blutungen zu zusätzlichen Komplikationen führen.

Die Erfindung stellt sich somit die Aufgabe, eine insbesondere als Klebstoffzusammensetzung zum Verkleben von biologischen Geweben geeignete Kombination bereitzustellen, welche einfach handhabbar und insbesondere sowohl für interne als auch für oberflächliche Wundversorgungen geeignet ist.

Diese Aufgabe wird gelöst durch eine Kombination, insbesondere zum Verkleben und/oder Fixieren von biologischen und/oder synthetischen Geweben, umfassend die Komponenten
a) mindestens ein, insbesondere ein, stickstofffunktionalisiertes Polysaccharid und
b) mindestens ein, insbesondere ein, endständig funktionalisiertes Oligolacton.

Der Verklebungsprozess der erfindungsgemäßen Kombination, bei der es sich bevorzugt um eine chirurgische Klebstoffzusammensetzung handelt, beruht auf einer Reaktion ihrer Komponenten. Mit besonderem Vorteil handelt es sich hierbei um eine in-situ Vernetzung der stickstofffunktionalisierten Polysaccharide mit den endständig funktionalisierten Oligolactonen. Durch die endständige Funktionalisierung stellen die Oligolactone der Kombination geeignete und insbesondere reaktive Vernetzungspartner für die Polysaccharide dar. Die Vernetzung beruht insbesondere auf kovalenten Bindungen. Daneben können auch physikalische Vernetzungsprozesse eine Rolle spielen. Durch die Reaktion zwischen den Komponenten der Kombination können Wundverschlüsse entstehen, deren Festigkeiten signifikant höher sind als beispielsweise bei einem Einsatz von herkömmlichen, klinisch erprobten Fibrinklebern. Die Haftung zum biologischen Gewebe wird darüber hinaus durch die Reaktion der endständigen funktionellen Gruppen der Oligolactone mit Gewebeproteinen erreicht.

In einer bevorzugten Ausführungsform ist das Polysaccharid ein natürlich vorkommendes Polysaccharid. Beispielsweise kann es sich bei dem Polysaccharid um Glykosaminoglykane (Mucopolysaccharide) handeln. Ein Beispiel für ein bevorzugtes natürlich vorkommendes Polysaccharid ist Hyaluronsäure. Die Hyaluronsäure kann beispielsweise ein Molekulargewicht von mindestens 20 kDa (kilo Dalton) besitzen.

Zusätzlich oder alternativ zu den vorstehend genannten Polysacchariden kann das erfindungsgemäß verwendete Polysaccharid von einem natürlich vorkommenden Polysaccharid abgeleitet sein. Typischerweise handelt es sich in diesem Fall um ein chemisch modifiziertes Polysaccharid. In dieser Variante beruht die Stickstofffunktionalisierung des Polysaccharids auf der chemischen Modifikation. Hierfür kommen grundsätzlich alle Polysaccharide in Betracht, die einer derartigen Funktionalisierung zugänglich sind.

Weiterhin ist es erfindungsgemäß möglich, dass das Polysaccharid der Kombination ein vollständig synthetisches Polysaccharid ist.

In einer besonders bevorzugten Ausführungsform ist das Polysaccharid ein Aminogruppen, insbesondere primäre Aminogruppen, tragendes Polysaccharid. Bevorzugt beträgt der Anteil an Monosaccharideinheiten im Polysaccharid, die eine Aminogruppe tragen, mindestens 30 %, vorzugsweise 80 %. Das Polysaccharid besitzt vorzugsweise ein Molekulargewicht von 10 bis 350 kDa, insbesondere ca. 200 kDa.

In einer weitergehenden Ausführungsform ist das Polysaccharid ein zumindest teilweise deacyteliertes Glykosaminoglykan. Die Verwendung eines entsprechenden Glykosaminoglykanderivats ist auch möglich. Als geeignete Polysaccharide kommen insbesondere zumindest teilweise deacyteliertes Chondroitinsulfat, Keratansulfat und/oder Hyaluronsäure in Frage.

In einer besonders bevorzugten Ausführungsform weist das Polysaccharid einen Deacytelierungsgrad zwischen 50 und 98 %, insbesondere zwischen 60 und 95 %, vorzugsweise zwischen 80 und 90 %, auf.

Besonders bevorzugt ist das Polysaccharid ein Chitosan. Die Verwendung von Chitosan ist wegen seiner blutstillenden (hämostatischen) und insbesondere antimikrobiellen Eigenschaften besonders vorteilhaft. Erfindungsgemäß kann es weiterhin vorgesehen sein, dass es sich bei dem Polysaccharid um ein Chitosanderivat handelt. Das Chitosan besitzt vorzugsweise ein Molekulargewicht zwischen 15 und 270 kDa. Das Chitosan weist bevorzugterweise einen Deacetylierungsgrad von ca. 86 % auf.

In einer weiteren Ausführungsform weist die Kombination mehrere verschiedene stickstofffunktionalisierte Polysaccharide auf. Diese können insbesondere in Form einer Mischung in der Kombination enthalten sein. Bezüglich geeigneter Polysaccharide wird auf die bisherige Beschreibung verwiesen.

Das im Rahmen der vorliegenden Erfindung eingesetzte endständig funktionalisierte Oligolacton dient vorzugsweise als Vernetzungsmittel. Als Oligolactone werden im Sinne der vorliegenden Erfindung "innere Ester" von Hydroxycarbonsäuren und somit im erweiterten Sinne auch Oligoglykolide, Oligolactide sowie gemischt zusammengesetzte Oligolactone, insbesondere auf der Basis von Glykolid und Lactid, bezeichnet. Diese können beispielsweise durch ringöffnende Polymerisation oder Oligomerisation hergestellt sein.

Zur Herstellung der Oligolactone werden bevorzugt Katalysatoren eingesetzt. Bei den Katalysatoren kann es sich grundsätzlich um Zinnverbindungen handeln. Zink- und/oder Eisenverbindungen sind aufgrund ihrer guten Biokompatibilität besonders bevorzugt.

Erfindungsgemäß weist das Oligolacton endständige Isocyanatgruppen auf. Dies ist besonders vorteilhaft, da eine entsprechende Kombination mit stickstofffunktionalisierten Polysacchariden und mit Isocyanatgruppen modifizierten Oligolactonen zu einer Klebeverbindung mit einer erhöhten Haftfestigkeit auf zu klebenden Substraten, insbesondere biologischen Geweben, reagiert.

Zur endständigen Funktionalisierung der Oligolactone werden diese vorzugsweise mit Diisocyanaten umgesetzt. Die Oligolactone der erfindungsgemäßen Kombination sind bevorzugterweise an ihrem Ende bzw. an ihren Enden mit einer aliphatischen Isocyanatgruppe modifiziert. Die Verwendung von aliphatischen Diisocyanaten, insbesondere Hexamethylendiisocyanat (HMDI), zur Funktionalisierung der Oligolactone ist besonders bevorzugt, da sich aus aromatischen Diisocyanaten cancerogene Diamine bilden können. Bei einer stöchiometrischen Umsetzung ist eine Aufreinigung nicht erforderlich. Gegebenenfalls kann jedoch eine Aufreinigung durchgeführt werden. Hierfür bietet sich abhängig von der Struktur sowie der Temperaturbeständigkeit des hergestellten Oligolactons beispielsweise eine Destillation an.

Das Oligolacton weist in einer weiteren Ausführungsform einen mehrwertigen, insbesondere mindestens zweiwertigen, Alkohol auf. Als geeignete Alkohole kommen insbesondere Ethylenglykol (1,2-Ethandiol) und/oder Glycerin (1,2,3-Propantriol) in Betracht.

Das Oligolacton ist vorzugsweise aus einem mehrwertigen Alkohol und Hydroxycarbonsäuren, insbesondere Glykol- und/oder Milchsäure, gebildet. Bevorzugt ist mindestens eine Hydroxylgruppe des mehrwertigen Alkohols mit Hydroxycarbonsäureeinheiten, insbesondere mit Milch- und/oder Glykolsäureeinheiten, verestert. Im Falle eines zweiwertigen Alkohols ist es besonders bevorzugt, wenn beide Hydroxylgruppen in veresterter Form vorliegen. Die Hydroxylgruppen des mehrwertigen Alkohols sind vorzugsweise, zumindest teilweise, mit einer Kette aus 1 bis 10, insbesondere 2 bis 5, Hydroxycarbonsäureeinheiten verestert. Mit zunehmender Anzahl der Einheiten steigt die Viskosität des Oligolactons sowie des im Rahmen einer Reaktion mit dem stickstofffunktionalisierten Polysaccharids erhaltenen Klebers an. Die im Rahmen dieser Ausführungsform beschriebenen Oligolactone aus mehrwertigem Alkohol und Hydroxycarbonsäureeinheiten tragen an der bzw. den endständigen Hydroxycarbonsäureeinheiten funktionelle Gruppen, welche die endständige Funktionalisierung des Oligolactons bilden.

Bevorzugt ist das Oligolacton ein Ethylenglykol-Oligolactid (EOL). Daneben können weitere Oligolactone, insbesondere Ethylenglykololigoglykolid (EOG ½, M = 294,2 g/mol), Pentaerythrytololigolactid (POL ¼, M = 712,6 g/mol), Glycerololigolactid (GOL 1/0,5, M = 164,1 g/mol) und Glycerololigolactid-co-glykolid (GOLG 1/1/3, M = 584,4 g/mol) verwendet werden.

Erfindungsgemäß kann es weiterhin vorgesehen sein, dass die Kombination mehrere verschiedene Oligolactone, insbesondere in Form einer Mischung, aufweist. Bezüglich der in Betracht kommenden Oligolactone wird auf die vorstehend gemachten Ausführungen verwiesen.

Sofern eine schnelle Reaktion der Komponenten der Kombination und damit eine schnelle Verklebung erwünscht ist, kann im Rahmen der erfindungsgemäßen Kombination als weitere Komponente ein Katalysator für die Reaktion zwischen den stickstofffunktionalisierten Polysacchariden und den endständig funktionalisierten Oligolactonen verwendet werden. Durch den Einsatz eines derartigen Katalysators kann die Reaktion der Hydroxylgruppen der Polysaccharide, im Falle von Aminogruppen tragenden Polysacchariden auch oder vor allem der Aminogruppen, mit den endständigen funktionellen Gruppen der Oligolactone stark beschleunigt werden. Eine katalytische Beschleunigung der Reaktion zwischen den Komponenten kann vor allem bei medizinischen Einsätzen sinnvoll sein. Beispielsweise kann bei besonders stark blutenden Wunden die Verwendung eines Katalysators wünschenswert sein, wohingegen beispielsweise beim Verkleben von Knochen eine langsamere Aushärtung der Kombination Sinn machen kann, um eine Einpassung mit der Möglichkeit einer späteren Korrektur zu ermöglichen.

Als geeignete Katalysatoren kommen insbesondere Amine, Amidine, vorteilhafterweise 2,3-Dimethyl-3,4,5,6-tetrahydropyrimidin, ein tertiäres Amin, vorteilhafterweise Triethylamin, Tributylamin, Dimethylbenzylamin, N-Methyl-, N-Ethyl-, N-Cyclohexylmorpholin, N, N, N', N'-Tetramethylethylendiamin, N, N, N', N'-Diaminoethylether, Bis-(dimethylamino-propyl)harnstoff, Dimethylpiperazin, 1,2-Dimethylimidazol, 1-Aza-bicyclo-(3,3,0)-octan und vorzugsweise 1,4-Diaza-bicyclo-(2,2,2)-octan und/oder ein Alkanolamin, wie Triethanolamin, Triisopropanolamin, N-Methyl- und N-Ethyl-diethanolamin und Di-methylethanolamin, vorzugsweise 1,4-Di-aza[2.2.2]bicyclo-octan (Dabco), in Frage.

Der Anteil des Polysaccharids in der Kombination beträgt in einer bevorzugten Ausführungsform 1 bis 80 Gew.-%, insbesondere 5 bis 70 Gew.-%. Der Oligolactonanteil in der Kombination beträgt vorzugsweise 20 bis 99 Gew.-%, insbesondere 30 bis 95 Gew.-%. Die Gewichtsprozentangaben beziehen sich auf die Gesamtmasse der Kombination ohne Lösungsmittel.

Durch Variation des Polysaccharid- und/oder Oligolactonanteils in der Kombination kann in besonders vorteilhafter Weise die Kleb- oder Haftkraft der aus der Kombination nach Reaktion ihrer Komponenten hervorgehenden Klebverbindung an den jeweiligen Verwendungszweck angepasst werden. So kann insbesondere durch einen größeren Oligolactonanteil ein höherer Vernetzungsgrad nach Mischen mit dem Polysaccharid ersielt werden. Durch eine größere Oligolactonmenge in der Kombination können auch verstärkt reaktive Hydroxyl- und/oder Aminogruppen von zu verklebenden Substraten, insbesondere biologischen Geweben, in die Vernetzung einbezogen werden, wodurch sich insgesamt die Klebkraft (Haft- bzw. Haltekraft) erhöht.

In einer zweckmäßigen Ausführungsform liegen das Polysaccharid und/ oder das Oligolacton in einem oder mehreren Lösungsmitteln dispergiert, vorzugsweise gelöst, vor. Bevorzugt handelt es sich bei dem Lösungsmittel um Wasser. Weiterhin können organische Lösungsmittel verwendet werden, die sinnvollerweise biologisch verträglich sind. Ein geeignetes Lösungsmittel ist Dimethylsulfoxid (DMSO). In einer bevorzugten Ausführungsform ist das Lösungsmittel ein Lösungsmittelgemisch aus DMSO und Wasser.

Das Oligolacton liegt in einer weiteren Ausführungsform in Form einer organischen Lösung, vorzugsweise einer DMSO-Lösung, insbesondere mit einem Oligolactonanteil zwischen 50 und 99 Gew.-%, insbesondere 70 und 90 Gew.-%, vorzugsweise von ca. 80 Gew.-%, bezogen auf das Gesamtgewicht der Lösung, vor.

Das Polysaccharid und das Oligolacton liegen getrennt voneinander vor. Vorteilhafterweise werden Polysaccharid und Oligolacton erst dann gemischt, wenn eine

Reaktion zwischen ihnen erwünscht ist. In einer zweckmäßigen Ausführungsform ist die erfindungsgemäße Kombination bereits in einer Applikationsvorrichtung enthalten. Beispielsweise können die Komponenten getrennt voneinander in einzelnen Kartuschen vorliegen, die bevorzugt in einer Mischeinheit plaziert sind. Vorzugsweise liegen das Polysaccharid und das Oligolacton getrennt voneinander in den Kammern einer Mehrkomponentenspritze, insbesondere einer Zweikomponenten- oder Doppelkammerspritze, etwa vom Typ Mixpack (Mixpack Systems AG, Rotkreuz, Schweiz), vor. Die Mehrkomponentenspritze besitzt zweckmäßigerweise einen Mischextruder zur Vermischung der zunächst getrennt voneinander vorliegenden Komponenten der Kombination.

Erfindungsgemäß ist es weiterhin bevorzugt, dass die Kombination in einer Sprühvorrichtung enthalten ist. Die Sprühvorrichtung kann ebenfalls mindestens zwei Kammern aufweisen. Dadurch können mit besonderem Vorteil die Komponenten der Kombination, insbesondere das Polysaccharid und das Oligolacton, getrennt voneinander bis zur eigentlichen Verwendung aufbewahrt werden.

In einer weiteren Ausführungsform wird eine Reaktion, vorzugsweise Vernetzung, der Komponenten nach ihrer Vermischung auf einem zu klebenden Substrat innerhalb eines Zeitraumes von < 100 Sekunden, insbesondere < 80 Sekunden, vorzugsweise < 60 Sekunden, initiiert bzw. gestartet.

In einer weitergehenden Ausführungsform liegt das Polysaccharid in Form einer wässrigen Dispersion, insbesondere wässrigen Lösung, vor. Das Polysaccharid liegt vorzugsweise in Form einer wässrigen Lösung mit einem Polysaccharidanteil von 2 bis 5 Gew.-%, vorzugsweise von 3 bis 4 Gew.-%, bezogen auf das Gesamtgewicht der Lösung, vor.

In einer weiteren Ausführungsform weist die Kombination Zusatz-, Hilfs- und/oder Füllstoffe auf. Bei den Zusatz- und/oder Hilfsstoffen kann es sich um antimikrobielle, insbesondere antibiotische, Wirkstoffe handeln. Bei den Füllstoffen kann es sich beispielsweise um Gelatine, Collagen und/oder Albumin, handeln. Die Verwendung von Gelatine und/oder Collagen hat den weiteren Vorteil, dass diese Verbindungen auch hämostatische Eigenschaften besitzen. Bevorzugte Füllstoffe, insbesondere zum Verkleben von Hartgewebe, sind aus der Gruppe Dicalciumphosphat, Tricalciumphosphat und Hydroxylapatit ausgewählt. Im Falle von Tricalciumphosphat kann es sich insbesondere um β-Tricalciumphosphat handeln. Carbonate, beispielsweise Calciumcarbonat, und Oxidmaterialien, beispielsweise Siliziumdioxid, sind weitere Beispiele für mögliche Füllstoffe. Schließlich kann es sich bei den Zusatz- bzw. Hilfsstoffen auch um Wachstumsfaktoren, entzündungshemmende, desinfizierende und/oder antimikrobielle Verbindungen handeln.

Erfindungsgemäß kann es durchaus vorgesehen sein, dass die Kombination aus dem stickstofffunktionalisierten Polysaccharid und dem endständig funktionalisierten Oligolacton besteht, gegebenenfalls noch aus mindestens einem Zusatz- bzw. Hilfsstoff. Diesbezüglich wird auf die bisherige Beschreibung verwiesen.

In einer vorteilhaften Ausführungsform liegen das Polysaccharid und/ oder das Oligolacton in sterilisierter Form vor. Die Komponenten der erfindungsgemäßen Kombination können beispielsweise getrennt von einander in einzelnen Kartuschen vorliegen. Die Kartuschen können vorteilhafterweise in einer Mischeinheit plaziert sein. Vorzugsweise sind die Komponenten steril verpackt, insbesondere in einer Mehrkomponentenspritze, bevorzugt mit einem aufgesetzten Mischextruder. Eine Sterilisierung der erfindungsgemäßen Kombination bzw. ihrer einzelnen Komponenten kann vorteilhafterweise ohne Strukturveränderung erzielt werden. Dies kann beispielsweise über eine Sterilfiltrierung von entsprechenden Lösungen vorgenommen werden. Vorzugsweise umfasst die Kombination eine Lösung des stickstofffunktionalisierten Polysaccharids, wobei die Lösung des Polysaccharids durch Dampfsterilisation sterilisiert ist. Die endständig funktionalisierten Oligolactone sind bevorzugt durch eine γ-Sterilisation in eine sterilisierte Form überführt. Grundsätzlich ist auch eine aseptische Abfüllung der Komponenten der erfindungsgemäßen Kombination möglich. Allerdings können sowohl die Dampfsterilisation als auch die Sterilisation über γ-Strahlung mit besonderem Vorteil an der bereits verpackt vorliegenden Kombination durchgeführt werden. Die erfindungsgemäße Kombination kann außerdem in konfektionierter Form vorliegen.

Die Kombination liegt gemäß einer besonders bevorzugten Ausführungsform als Klebstoffzusammensetzung vor.

Die Kombination eignet sich besonders zum Verkleben und/oder Fixieren von biologischen Geweben, wobei es sich bei den zu verklebenden Geweben bevorzugt um Hart- und/oder Weichgewebe handelt. Bei dem zu verklebenden Hartgewebe kann es sich insbesondere um Knochengewebe, beispielsweise in Form von Knochenhöhlen, und/oder Zahnextraktionen handeln. Die zu klebenden Weichgewebe können insbesondere Organe, Blutgefäße und/oder parenchymales Gewebe sein. Als mögliche Organe kommen insbesondere Leber, Niere, Milz und/oder Lunge in Betracht.

Die erfindungsgemäße Kombination kann weiterhin zum Fixieren und/ oder Abdichten von Implantaten, insbesondere Herniennetzen, Gefäßprothesen, Katheter, Stents und/oder Ersatzmaterialien für die Dura Mater, verwendet werden. Eine Möglichkeit ist beispielsweise die Fixierung eines Herniennetzes an der Bauchwand. Bei dem zu fixierenden Dura Mater Ersatzmaterial kann es sich insbesondere um ein Rinderperikard handeln. Durch die Verwendung der erfindungsgemäßen Kombination ist ein Annähen des Ersatzmaterials nicht erforderlich, wodurch Zeit und Behandlungskosten eingespart werden können.
Eine weitere vorteilhafte Anwendung der Kombination ist die Abdichtung von Anastomosen und/oder Wundverbänden (Patchen) an Hohlorganen, beispielsweise Blutgefäßen. Die erfindungsgemäße Kombination kann ferner zur Fixierung von sogenannten Drug-Delivery-Devices und insbesondere von porösen Trägerstrukturen und/oder Membranen für den potenziellen Einsatz in der regenerativen Medizin (tissue engineering) verwendet werden.

Ein weiteres Anwendungsgebiet der erfindungsgemäßen Kombination ist die Wundsanierung von insbesondere infizierten Wunden. Grundsätzlich ist ein Einsatz der Kombination in der plastischen, rekonstruktiven und/oder kosmetischen Chirurgie denkbar, insbesondere zur Vermeidung von Narbenbildung, die vor allem beim Nähen von Wunden verursacht werden.

In einer besonders bevorzugten Ausführungsform wird die Kombination zum Verschluß oder zur Versiegelung bzw. Abdichtung von Flüssigkeits- und/oder Luftleckagen im menschlichen und/oder tierischen Körper verwendet. Beispielsweise kann die Kombination zur Abdichtung von Lungenleckagen, Darmleckagen, Blasenleckagen, Harnleiterleckagen, Herzbeuteln, Darm- und/oder Gefäßanastomosen verwendet werden. Darüber hinaus eignet sich die Kombination auch für eine Abdichtung von Stichkanalblutungen in der Gefäßchirurgie sowie zur Abdichtung bei arteriellen Bypassoperationen. Die durch die Vernetzung der Kombination entstehende Klebeverbindung weist bevorzugt elastische Eigenschaften auf, was insbesondere bei sich stark ausdehnenden Geweben, beispielsweise dem Lungengewebe, aber auch bei pulsierenden Geweben, beispielsweise Blutgefäßen, von Vorteil ist.

In einer weiteren möglichen Ausführungsform kann die Kombination zur Versiegelung von minimal invasiven Blutungen eingesetzt werden. Die Applikation erfolgt zweckmäßigerweise über einen Trokar. Eine Verwendung der Kombination zur Füllung von Zahnextraktionshöhlen und/oder Knochendefekten kann erfindungsgemäß ebenfalls vorgesehen sein. Zusätzlich oder alternativ zu den bisher beschriebenen Verwendungsmöglichkeiten ist es erfindungsgemäß denkbar, dass die Kombination zur Fixierung von Muskeln, Bändern und/oder Sehnen an Knochen eingesetzt werden kann. Die Kombination kann außerdem zum Verkleben von Wundrändern verwendet werden. Auch die Versorgung von chirurgisch gesetzten Wunden, beispielsweise nach einer Tumorentfernung, ist mit der erfindungsgemäßen Kombination möglich.

Des Weiteren ermöglicht die erfindungsgemäße Kombination auch eine Anwendung in besonders schwierigen Fällen, insbesondere bei der Versorgung von chronischen Wunden. Bei solchen Wunden entsteht nach der notwendigen chirurgischen Versorgung eine starke Zugspannung, die die Wundheilung gewöhnlich erschwert. Die hohe Festigkeit des erfindungsgemäßen Wundverschlusses ermöglicht auch in diesen Fällen eine feste Abdichtung und verhindert insbesondere Wundinfektionen. Nach Abschluss der Vernetzungsreaktion können auf den so verschlossenen Wunden beispielsweise Kompressiv-Bandagen angewandt werden, um eine Ödembildung zu verhindern.

Die vorliegende Erfindung umfasst auch eine Verbindung, bei der die Komponenten der erfindungsgemäßen Kombination miteinander vermischt sind und die stickstofffunktionalisierten Polysaccharide zumindest teilweise über die endständig funktionalisierten Oligolactone miteinander vernetzt sind. Wie bereits erwähnt, kann die Vernetzung insbesondere auf kovalenten Bindungen beruhen, wobei allerdings auch eine physikalische Vernetzung in Betracht kommt. Bevorzugt sind auch reaktive funktionelle Gruppen, insbesondere Hydroxyl- und/oder Aminogruppen, von zu verklebenden Substraten in die Vernetzung der erfindungsgemäßen Verbindung einbezogen. Die Verbindung weist vorteilhafterweise eine gute Bioverträglichkeit auf.

Gegenstand der Erfindung ist somit auch eine Klebstoffzusammensetzung, die die erfindungsgemäße Kombination, d. h. die Komponenten a) und b) aufweist. Mit anderen Worten betrifft die vorliegende Erfindung auch eine Klebstoffzusammensetzung, insbesondere zur Verklebung und/oder Fixierung von biologischen und/oder synthetischen Geweben, umfassend die Komponenten
a) mindestens ein, insbesondere ein, stickstoffunktionalisiertes Polysaccharid
b) mindestens ein, insbesondere ein, endständig funktionalisiertes Oligolacton.

Bezüglich weiterer Einzelheiten und Merkmale wird daher auf die bisherige und die noch folgende Beschreibung Bezug genommen.

Ein Vorteil des durch Reaktion der erfindungsgemäßen Kombination entstehenden Klebers ist seine biologische Abbaubarkeit. Durch die Vernetzung der stickstoffunktionalisierten Polysaccharide über die endständig funktionalisierten Oligolactone entsteht eine sehr feste Verklebung, die im Verlauf des Heilungsprozesses idealerweise resorbiert werden kann. Hierbei sind insbesondere die anfängliche Härtungsphase, verbunden mit hohen Klebeigenschaften, und die zeitabhängig langsame Resorption im Körper bei fortgeschrittenen Heilungsprozessen von besonderem Vorteil. Bevorzugt wird der Kleber während eines Zeitraums von etwa 1 Jahr resorbiert (biologisch abgebaut), bevorzugt während eines Zeitraums von 2 bis 6 Monaten. Der Mechanismus der Resorption kann beispielsweise auf hydrolytischen und/oder enzymatischen Prozessen beruhen. Insbesondere können die stickstofffunktionalisierten Polysaccharide gespalten und einzelne Fragmente davon abtransportiert und ausgeschieden werden. Alternativ können die Fragmente, insbesondere Monosaccharide, auch in Zellen eingeschleust werden.

Weiterhin umfasst die vorliegende Erfindung ein medizintechnisches Produkt, insbesondere zum Verkleben und/oder Fixieren von biologischen und/oder synthetischen Geweben, das die erfindungsgemäße Kombination aufweist. Das medizintechnische Produkt ist vorzugsweise ein Implantat, beispielsweise ein Herniennetz. Weiterhin kann es sich bei dem medizintechnischen Produkt um Ersatzmaterialien, insbesondere zum Verschluss und/oder zur Abdichtung der Dura Mater, handeln. Das medizintechnische Produkt kann zudem ein Dentalimplantat, insbesondere zur Füllung von Zahnextraktionshöhlen, sein. Erfindungsgemäß ist es ebenso möglich, dass das medizintechnische Produkt ein Knochenimplantat, insbesondere zur Behandlung und/oder Füllung von Knochendefekten, ist.

Vorzugsweise handelt es sich bei dem medizintechnischen Produkt um ein Hämostyptikum (blutstillendes Mittel bzw. Hämostatikum). In dieser Ausführungsform eignet sich das medizintechnische Produkt vorzugsweise zur Versiegelung von inneren blutenden und äußeren blutenden Wunden. Die erfindungsgemäße Kombination bildet nach Vermischen seiner Komponenten mit besonderem Vorteil eine pastöse und insbesondere klebrige Masse, welche beispielsweise nach Applikation auf einen zu versorgenden Wundbereich eine Art physikalische Barriere für Körperflüssigkeiten, insbesondere Blut und/oder Exsudat, darstellt. Dadurch wird mit besonderem Vorteil ein Abfließen des Blutes aus dem Wundmilieu verhindert, wodurch Blutplättchen und insbesondere Gerinnungsfaktoren angereichert werden. Dies führt vorteilhafterweise zu einer beschleunigten Blutstillung im Wundbereich.

In einer bevorzugten Ausführungsform handelt es sich um ein medizintechnisches Produkt zum Verkleben von Weichgewebe. Zum Weichgewebe gehören, wie bereits erwähnt, hauptsächlich Organe, Blutgefäße und/oder parenchymales Gewebe. Besonders bevorzugt ist der Einsatz des medizintechnischen Produktes zum Kleben von Rupturen verschiedener Organe, beispielsweise der Leber, der Niere und/oder der Milz. Des Weiteren kann das medizintechnische Produkt zum Verschluss von Leckagen, insbesondere Lungenleckagen, verwendet werden. Die durch Vermischen der Komponenten der erfindungsgemäßen Kombination entstehende pastöse Masse besitzt vorteilhafterweise eine hohe Elastizität, was insbesondere bei sich stark ausdehnenden Geweben von Vorteil ist.

In einer weiteren Ausführungsform eignet sich das medizintechnische Produkt zum Kleben von Hartgewebe, insbesondere von Knochen. Für das Kleben von Knochen sowie für eine schnelle Integration von Implantaten, insbesondere im Bereich der Knochen, ist die Akzeptanz des implantierten Materials durch die Knochenzellen von besonderer Wichtigkeit. Dies trifft selbstverständlich auch auf den verwendeten Kleber zu. Durch die schnelle Besiedelung mit Osteoblasten und deren anschließender Ausreifung kann eine Knochenneubildung gefördert und der Heilungsprozess zum Teil erheblich verkürzt werden. In dieser Ausführungsform ist das medizintechnische Produkt daher zweckmäßigerweise ein Implantat mit einer mikrostrukturierten Oberfläche, wobei die erfindungsgemäße Kombination diese Oberflächenstruktur vorteilhafterweise erhält und insbesondere die Knochenneubildung unterstützt. Des Weiteren ist auch eine Anwendung bei komplizierten Brüchen möglich. So können beispielsweise mit Hilfe des erfindungsgemäßen medizintechnischen Produktes Knochensplitter wieder eingefügt werden. Das medizintechnische Produkt eignet sich weiterhin bevorzugt zum Fixieren von Trümmerfrakturen, beispielsweise im Gesichts-Schädel-Bereich oder der Extremitäten, insbesondere bei Radiusköpfchenfraktur.

Die vorliegende Erfindung bezieht sich weiterhin auf einen Kit, insbesondere zum Kleben, umfassend mindestens zwei, vorzugsweise zwei, Behältnisse. Das eine Behältnis enthält ein stickstofffunktionalisiertes Polysaccharid und das andere Behältnis ein endständig funktionalisiertes Oligolacton. Bei den Behältnissen des erfindungsgemäßen Kits handelt es sich vorzugsweise um die Kammern einer Mehrkomponentenspritze, insbesondere einer Zweikomponenten- oder Zwillingsspritze. Die Spritze weist zweckmäßigerweise einen Mischextruder zum Mischen des Polysaccharids und des Oligolactons auf. Weiterhin können die Behältnisse auch Bestandteil eines Sprühapplikators oder einer Sprühvorrichtung sein. Bezüglich weiterer Einzelheiten und Merkmale, insbesondere hinsichtlich des verwendeten Polysaccharids und/oder Oligolactons wird auf die bisherige Beschreibung verwiesen.

In einem weiteren Aspekt betrifft die Erfindung ein in-vitro Verfahren zum Kleben, bei dem die Komponenten der efindungsgemäßen Kombination miteinander und mit mindestens einem, bevorzugt mindestens zwei, zu verklebenden Substrat; das reaktive funktionelle Gruppen aufweist, in Kontakt gebracht werden. Bei den funktionellen Gruppen der zu (ver)klebenden Substrate handelt es sich vorzugsweise um Hydroxyl- und/oder Aminogruppen. Bei dem Klebeverfahren erfolgt die Verbindung der Substrate bevorzugt durch eine Vernetzung von Hydroxyl- und/oder gegebenenfalls Aminogruppen der stickstofffunktionatisierten Polysaccharide und der Substrate durch die Oligolactone.

Dabei ist bevorzugt mindestens eines der Substrate ein Organ, Gewebe, Zahnkompartiment und/oder Knochen. Als Zahnkompartimente kommen insbesondere Schmelz und/oder Dentin in Betracht. Vorzugsweise sind die Substrate Wundränder, die verklebt werden sollen. Alternativ kann es sich jedoch auch zum Beispiel um miteinander zu verktebende anorganische oder organische Substrate handeln, die keine Gewebe sind, etwa Kunststoff- oder Keramikoberflächen. Das Klebeverfahren kann insbesondere in einem wässrigen Milieu durchgeführt werden.

Weiterhin ist es möglich, als Substrate nicht nur makroskopische Substrate einzusetzen, sondern auch andere Reagenzien, welche vorzugsweise Hydroxyl- und/oder Aminogruppen enthalten. Diese können untereinander, aber auch mit festen Trägern, verbunden sein. Denkbare Substrate sind beispielsweise niedermolekulare Verbindungen, insbesondere Peptide, und/oder Zellen. Bezüglich weiter in Frage kommende Substrate wird auf die bisherige Beschreibung verwiesen.

Die vorliegende Erfindung bezieht sich schließlich noch auf die Verwendung der erfindungsgemäßen Kombination zur Herstellung eines medizintechnischen Produktes. In Bezug auf weitere Einzelheiten und Merkmale zu dem medizintechnischen Produkt wird auf die bisherige Beschreibung verwiesen.

Durch die Erfindung wird mit besonderem Vorteil ein Kombinationspräparat bereitgestellt, welches insbesondere auf medizinischem Gebiet vielfältig verwendbar ist. Die erfindungsgemäße Kombination eignet sich, wie bereits erwähnt, insbesondere zur Verklebung von äußeren und inneren Wunden. Die Kombination eignet sich auch zur Versorgung von stark blutenden und/oder von chronifizierten Wunden. Mit besonderem Vorteil kann die Viskosität und Fließfähigkeit der erfindungsgemäßen Kombination, abhängig beispielsweise von der Länge der zu verklebenden Wunde und insbesondere der Wundspalttiefe bzw. den zu verklebenden Substraten angepasst werden. Dies kann beispielsweise durch die Art der Polysaccharide und/oder der Oligolactone, insbesondere durch deren Länge (Anzahl der veresterten Hydroxycarbonsäureeinheiten), beeinflusst werden. Diese Parameter können zusätzlich durch die Wahl eines Lösungsmittels oder eines Lösungsmittelgemisches beeinflusst werden, wobei die Art des Lösungsmittels ebenfalls von Bedeutung ist. Daneben oder alternativ dazu können auch Zusatzstoffe, beispielsweise Thixotropiemittel, insbesondere nanodisperse Calciumphosphate (z.B. β-Tricalciumphosphat) oder nanodisperse Kieselsäuren zur Anpassung der Viskosität und Fließfähigkeit verwendet werden.

Weitere Merkmale und Vorteile der Erfindung ergeben sich aus der nachfolgenden Figurenbeschreibung, der Beispiele in Verbindung mit den Unteransprüchen. Hierbei können die einzelnen Merkmale jeweils für sich allein oder zu mehreren in Kombination miteinander verwirklicht sein.

In den Figuren ist Folgendes dargestellt:
- Figur 1:: Reaktionsschemata zur Herstellung von EOL-NCO,
- Figur 2:: Ausschnitt aus einem Chitosanmolekül,
- Figur 3:: dynamische Untersuchungen zur Bestimmung des Gelpunk- tes eines Fibrinklebers (Auftragung des Speichermoduls G' [Pa] und Verlustmoduls G" [Pa] gegen die Zeit t [s]),
- Figur 4:: dynamische Untersuchungen zur Bestimmung des Gelpunk- tes an einer erfindungsgemäßen Kombination (EOL-NCO (γ-sterilisiert)/Protasan UPCI 213 (wasserdampfsterilisiert), Auftragung des Speichermoduls G' [Pa] und Verlustmoduls G" [Pa] gegen die Zeit t [s]),
- Figur 5:: Klebefestigkeit verschiedener Klebersysteme, einschließlich von erfindungsgemäßen Kombinationen, auf bovinem Mus- kelgewebe bei Raumtemperatur (auf der Ordinate ist die Klebefestigkeit [N/m²] und auf der X-Achse die untersuchten Klebersysteme aufgetragen),
- Figur 6:: Klebefestigkeit verschiedener Klebersysteme, einschließlich von erfindungsgemäßen Kombinationen, auf bovinem Mus- kelgewebe bei 37 °C (jeweils n = 12 Versuche, auf der Ordi- nate ist die Klebefestigkeit [N/m²] aufgetragen, auf der Ab- szisse sind die untersuchten Klebersysteme aufgetragen),
- Figur 7:: Klebefestigkeit verschiedener Klebersysteme, einschließlich einer erfindungsgemäßen Kombination, auf bovinem Mus- kelgewebe bei 37°C (auf der Ordinate ist die Klebefestigkeit [N/m²], und auf der Abszisse sind die untersuchten Kleber- systeme aufgetragen, jeweils n = 12 Versuche),
- Figur 8:: Klebefestigkeit einer erfindungsgemäßen Kombination auf einem Polyurethanhartschaum bei 37 °C nach 4 h, 24 h, 48 h und 72 h (auf der Ordinate ist die Klebefestigkeit [kN/m²] und auf der Abszisse die Zeit [h] aufgetragen, jeweils n = 12 Versuche).

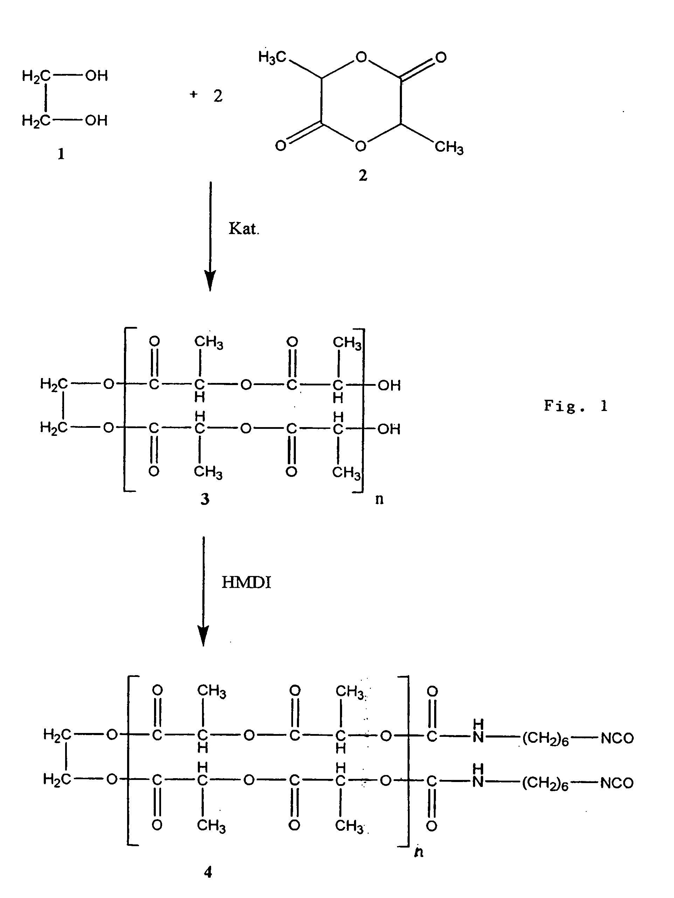

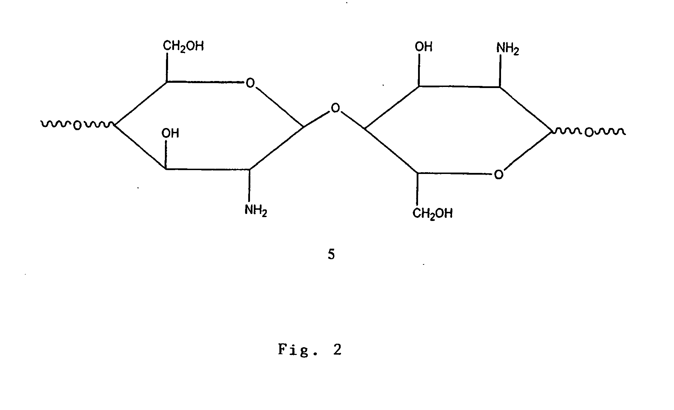

In Figur 1 ist die Herstellung von Ethylenglykol-Oligolactid (EOL) 4 gezeigt, das an seinen endständigen Milchsäureeinheiten mit Isocyanatgruppen modifiziert ist (EOL-NCO). Die Synthese erfolgt ausgehend von Ethylenglykol 1 und cyclischen Lactiden 2, wobei die Hydroxylgruppen von 1 mit den Lactiden 2 durch eine ringöffnende Oligomerisierung, gegebenenfalls auch ringöffnende Polymerisierung, verestert werden. Zur terminalen Funktionalisierung des hieraus hervorgehenden Ethylenglykol-Oligolactids (EOL) 3 werden dessen endständige Hydroxylgruppen mit Hexamethylendiisocyanat (HMDI) unter Ausbildung des endständig funktionalisierten Oligolactids 4 modifiziert. Das Oligolactid 4 kann nun als Vernetzungsmittel für stickstofffunktionalisierte Polysaccharide, beispielsweise für ein Chitosan 5, dessen Aufbau in Figur 2 schematisch angedeutet ist, und zu klebenden Substraten verwendet werden. In Figur 1 kann n beispielsweise 1, 2, 3, 4 oder 5 sein.

### Beispiel 1: Synthese von Ethylenglykololigolactid mit endständigen Isocyanatgruppen (EOL-NCO)

Es wurden 33 g (0.094 mol) des Oligoesters Ethylenglykololigolactid (EOL) mit 31.6 g (0.188 mol) Hexamethylendiisocyanat (HMDI) während 4 Stunden bei 50 °C unter Argon umgesetzt. Das Reaktionsgemisch wurde danach bei 50 °C während 2 Tage gerührt. Man erhielt ein farbloses, wasserunlösliches, viskoses Öl als Reaktionsprodukt, das im IR-Spektrum eine starke Isocyanatbande bei 2266 cm⁻¹ zeigte. Eine zusätzlich durchgeführte NMR-Spektroskopie bestätigte das Vorliegen des Reaktionsproduktes EOL-NCO.

### Beispiel 2: Rheologische Untersuchungen zur Bestimmung der Viskosität und des Gelpunktes

### 2.1 Viskosität

Die rheologischen Untersuchungen zur Viskosität des Kleberpräpolymers EOL-NCO und des verwendeten Vernetzungspartners Protasan UPCI 213 (medical grade, M_{w}= 270 kDa, 4 %ig in H₂O, Firma FMC Biopolymers Drummen Norwegen) wurden am Rheometer Rheostress^{®} 1 der Firma Thermo Haake unter folgenden Meßbedingungen durchgeführt: Meßsystem Platte-Platte (Durchmesser 20 mm), Meßspalt 1 mm, Oszillationsversuch, Meßmodus CS (controlled stress = Schubspannungsvorgabe), Meßverfahren Zeit-Sweep, Versuchsparameter: Schubspannung T = 5 Pa; Frequenz f = 1 Hz.; Temperatur T = 37 °C. Das aufgetragene Volumen der jeweils zu messenden Kleberkomponenten betrug 700 µl. Für jede Kleberkomponente wurde die komplexe dynamische Viskosität |η*| = [Pa·s] ermittelt. Die in Tabelle 1 dargestellten Werte für |η*| nach 300 s und 600 s sind Mittelwerte, die sich aus jeweils 3 Einzelmessungen ergeben.

**Tabelle 1: Komplexe dynamische Viskosität der untersuchten Kleberkomponenten**

| | **Viskosität \|η*\| [Pa·s]** | |
|---|---|---|
| **Kleberkomponente** | bei t = 300 s | bei t = 600 s |
| EOL-NCO (EOUHMDI, 1/2 n/n) | 261.7 ± 9.9 | 250.1 ± 8.8 |
| EOL-NCO (EOUHMDI, 1/3 n/n) | 2.0 ± 0.02 | 2.0 ± 0.03 |
| EOL-NCO (EOUHMDI, 1/2 n/n)/ DMSO (89/11 w/w) | 36.7 ± 3.0 | 35.7 ± 2.9 |
| EOL-NCO (EOUHMDI, 1/2 n/n)/ DMSO (85/15 w/w) | 5.8 ± 0.2 | 5.8 ± 0.2 |
| EOL-NCO (EOUHMDI, 1/2 n/n)/ DMSO (85/15 w/w), γ-sterilisiert (> 25 kGy) | 8.6 ± 0.4 | 8.8 ± 0.7 |
| Protasan UPCI 213 (M_{w} = 270 kDa, 4 %ig in H₂O) | 6.7 ± 0.3 | 7.8 ± 0.5 |
| Protasan UPCI 213 (M_{w} = 270 kDa, 4 %ig in H₂O), dampfsterilisiert (121 °C, 20 min) | 5.1 ± 0.5 | 6.6 ± 0.6 |
| Protasan UPCI 213 (4 %ig in H₂O), γ-sterilisiert (> 25 kGy) | < 0.5 | < 0.5 |

Da bei der Applikation über Zweikammerspritzen die beiden Kleberkomponenten zur optimalen Durchmischung eine ähnliche Viskosität aufweisen sollten, wurde die Viskosität der Vernetzungspartner an die des EOL-NCO, angemischt mit DMSO, angepaßt. Nach γ-Sterilisation wurde eine starke Verringerung der Lösungsviskosität in Folge von Kettenabbau beobachtet. Mit EOL-NCO (EOUHMDI, 1/2 n/n)/DMSO (85/15 w/w) vergleichbare Viskositäten wurden mit Protasan UPCI 213 nach Wasserdampfsterilisation erreicht. Deshalb wurden in den weiteren Untersuchungen nur wasserdampfsterilisierte Protasan UPCI 213-Lösungen verwendet.

### 2.2 Gelpunkt

Um die Verarbeitbarkeit der flüssigen Kleberkomponenten als Zweikomponentensystem beurteilen zu können, wurde der Gelpunkt mit dem Rheometer Rheostress^{®} 1 der Thermo Haake bestimmt. Der Gelpunkt ist als Sol-/Gel-Übergangspunkt definiert, bei dem der Speichermodul (elastischer Anteil) G' gleich dem Verlustmodul G" (viskoser Anteil) ist. Er kennzeichnet den Übergang vom Flüssigkeitsverhalten (G" > G') zum gelartigen bzw. Feststoffverhalten (G' > G"). Zur Gelpunktermittlung wurden folgende Meßbedingungen eingesetzt: Meßsystem Platte-Platte (Durchmesser 20 mm), Meßspalt 1 mm, Oszillationsversuch, Meßmodus CD (controlled deformation = Deformationsvorgabe), Meßverfahren Zeit-Sweep, Versuchsparameter: Deformation γ = 0,1; Frequenz f = 5 Hz; Temperatur T = 37 °C; Dauer t = 2.000 s, Auftragsvolumen: 1 ml.

Der Gelpunkt wurde anhand des Schnittpunktes der Module aus 5 Messungen nach Mischung von EOL-NCO (EOUHMDI 1/2 n/n)/DMSO (85/15 w/w, γ-sterilisiert > 25 kGy) und Protasan UPCI 213 (medical grade M_{w}= 270 kDa, 4 %ig in H₂O, Wasserdampf sterilisiert 121 °C, 20 min) bestimmt und dem Fibrinkleber (Tissucol duo S Immuno, Firma Baxter Deutschland GmbH, Unterschleißheim) gegenübergestellt.

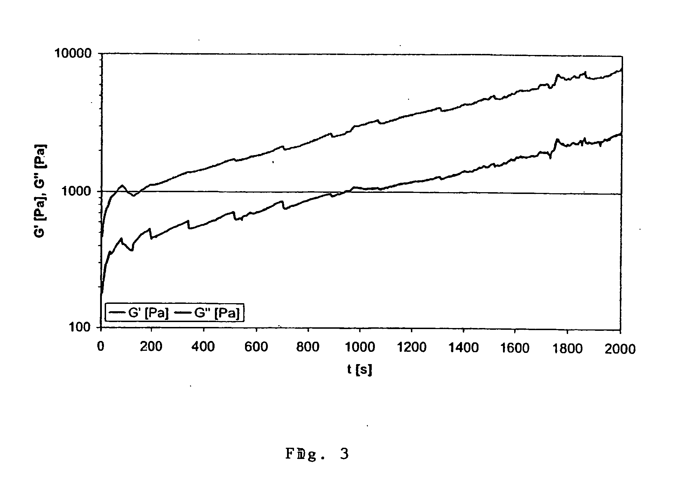

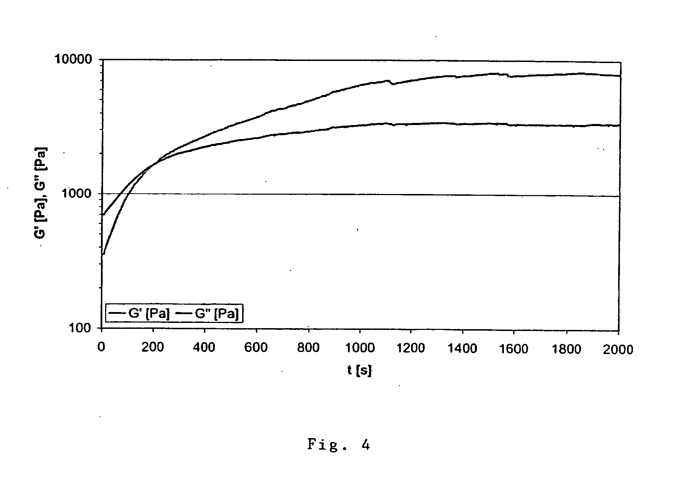

Die Messungen ergaben, dass der Fibrinkleber seinen Gelpunkt sofort erreicht und meßtechnisch mit dem Rheometer nicht erfaßbar war. Dies verdeutlichte sich durch die Tatsache, daß gleich zu Meßbeginn G' > G" war (Fig. 3). Der Sol-/Gelübergang von EOL-NCO (EOL/HMDI 1/2 n/n)/ DMSO (85/15 w/w)/Protasan UPCI 213 (M_{w} = 270 kDa, 4 %ig in H₂O) hingegen lag bei 196,4 s ± 40,1 s (Fig. 4). Die verzögerte Topf- bzw. Gelzeit der erfindungsgemäßen Kombination gegenüber Fibrinkleber hat für den Anwender den Vorteil, dass nach Applikation der Kombination noch eine ausreichende Verarbeitungszeit zur Verfügung steht, beispielsweise zur Fixation der zu verklebenden Gewebeteile, Knochenfragmente und/oder zur Positionierung bzw. Repositionierung von Defektdeckungsmaterialien. Die hohe Viskosität der Einzelkomponenten der Kombination verhindert, daß die Komponenten aus dem Wund- bzw. vom Applikationsort wegfließen oder im Falle von stark blutenden oder stark exsudierenden Wunden aus dem Wundbereich weggespült werden.

### Beispiel 3:

In 100 ml Wasser wurden ca. 4g Protasan UPCI 213 (FMC Biopolymers, Drummen Norwegen) gelöst. Die Lösung wurde anschließend bei ca. 121 °C während 20 Minuten mittels Dampfsterilisierung sterilisiert. Nach der Sterilisierung wurde das Molekulargewicht des Chitosans mit Hilfe von GPC (Gas Pressure Cromatography) bestimmt. Das Molekulargewicht betrug ca. 216 kDa. Die Viskosität der sterilen 4 Gew.-%igen Protasanlösung betrug ca. 1,99 Pas.

### Beispiel 4:

Eine Mischung aus Ethylenglykololigolactid, welches an seinen Enden durch Reaktion mit Hexamethylendiisocyanat (HMDI) modifiziert ist, und DMSO wurde mittels γ-Strahlung (> 25 kGy) sterilisiert. Dabei konnte anhand von NMR-Untersuchungen gezeigt werden, dass die chemische Struktur des mit HMDI endgekappten Ethylenglykololigolactids erhalten blieb.

### Beispiel 5: Prüfung der Festigkeit der Klebefuge an Weichgewebe

Die mechanische Testung der Klebefuge erfolgte unter einachsiger Zugbeanspruchung an der Universalprüfmaschine Zwick BZ2.5/TN1S (Zwick GmbH & Co. KG, Deutschland; Kraftmeßdose 50 N). Zur Prüfung der Klebeverbindungen wurden die Weichgewebeteile, im speziellen Teile bovinen Muskelgewebes, in formschlüssigen Probeneinspannungen aus Polymethylmethacrylat (PMMA) fixiert. Zur Schaffung einer vergleichbaren Bezugsfläche für die Klebung wurden die eingespannten Weichgewebeproben auf eine durchschnittliche Größe von 230 mm² zugeschnitten. Anschließend wurden nach manueller Anmischung in einem Vial 200 µl des zu untersuchenden Klebersystems auf das Gewebe aufgetragen. Die präparierten Weichgewebestücke wurden in der Universalprüfmaschine bei einem definierten Druck von 2 N 10 Minuten bei Raumtemperatur (23 °C) bzw. 37 °C gehalten und anschließend mit einer Prüfgeschwindigkeit von 10 mm/min unter Messung des Kraft-Weg-Verhältnisses auseinandergezogen und unter Berücksichtigung der Bezugsfläche die Klebefestigkeit ermittelt.

### 5.1 Durchführung der Klebereaktion für die Weichgewebeklebung bei Raumtemperatur (23 °C) nach manueller Anmischung

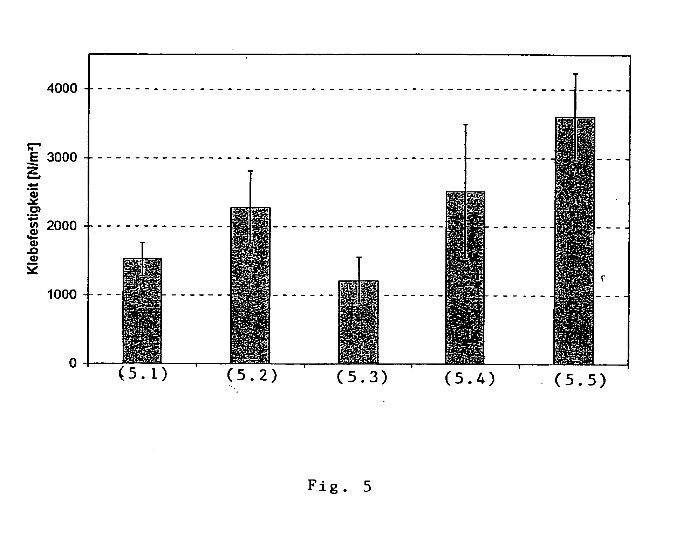

Folgende Klebersysteme wurden in Reihenuntersuchungen bei Raumtemperatur auf ihre Klebefestigkeit [N/m²] untersucht: (5.1) ohne Kleberzusatz; (5.2) Fibrin (Tissucol duo S Immuno, Firma Baxter Deutschland GmbH, Unterschleißheim); (5.3) EOL-NCO (EOL-HMDI 1/2 n/n)/DMSO (85/15 w/w, Komponente 1) und H₂O (Komponente 2); (5.4) EOL-NCO (EOUHMDI 1/3 n/n, Komponente 1) und Protasan UPCI 213 (M_{w} = 270 kDa g/mol, 4 %ig in H₂O, Komponente 2); (5.5) EOL-NCO (EOUHMDI 1/2 n/n)/DMSO (85/15 w/w, Komponente 1) und Protasan UPCI 213 (M_{w} = 270 kDa, 4 %ig in H₂O, Komponente 2). Die Mittelwerte der Klebefestigkeiten aus jeweils 12 Einzelversuchen sind in der Figur 5 aufgetragen.

### 5.2 Durchführung der Klebereaktion für die Weichgewebeklebung bei 37 °C nach manueller Anmischung

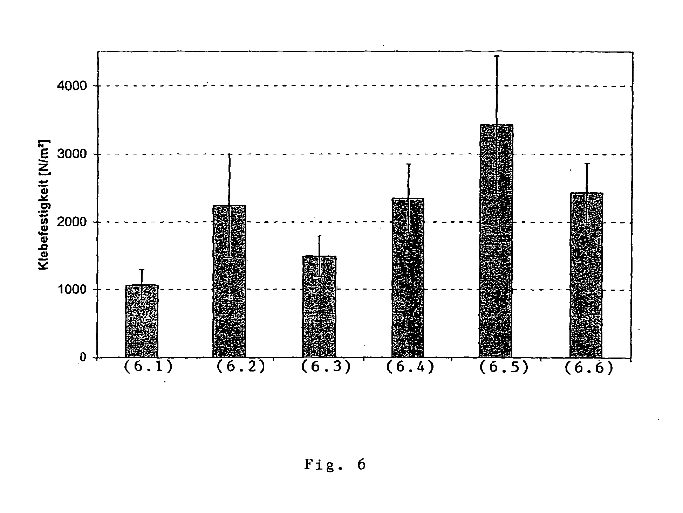

Um die Klebewirkung bei Körpertemperatur untersuchen zu können, wurden weitere Tests bei 37 °C durchgeführt. Hierzu wurden die in den PMMA-Halterungen eingespannten und in Folie verpackten Weichgewebestücke vor den Versuchen durch Lagerung im Wärmeschrank bei 37 °C eine Stunde temperiert. Des Weiteren wurde die Universalprüfmaschine mit einer temperierbaren Klimakammer (Δθ = 0,5 °C) versehen, so dass der Zugversuch bei 37 °C stattfinden konnte. Somit wurden die bereits bei Raumtemperatur durchgeführten Versuchsreihen zur Ermittlung der Haftfestigkeit auf Weichgewebe bei 37 °C wiederholt: (6.1) ohne Kleberzusatz; (6.2) Fibrin (Tissucol duo S Immuno, Firma Baxter Deutschland GmbH, Unterschleißheim); (6.3) EOL-NCO (EOUHMDI 1/2 n/n)/DMSO (85/15 w/w, Komponente 1) und H₂O (Komponente 2); (6.4) EOL-NCO (EOL/HMDI 1/3 n/n, Komponente 1) und Protasan UPCI 213 (M_{w} = 270 kDa, 4 %ig in H₂O, Komponente 2); (6.5) EOL-NCO (EOUHMDI 1/2 n/n)/DMSO (85/15 w/w, Komponente 1) und Protasan UPCI 213 (M_{w} = 270 kDa, 4 %ig in H₂O, Komponente 2). Zusätzlich wurde der Gehalt des Lösungsmittels DMSO verändert, so dass die Klebervariante (6.6) mit EOL-NCO (EOL/HMDI 1/2 n/n)/DMSO (89/11 w/w, Komponente 1) und Protasan UPCI 213 (4 %ig in H₂O, Komponente 2) resultierte. Die Mittelwerte der einzelnen Versuchsreihen (n = 12) sind in der Figur 6 dargestellt.

### Beispiel 5.3: Durchführung der Klebereaktion für die Weichgewebeklebung bei 37 °C unter Verwendung von Zweikammerspritzen mit sterilisierten Kleberkomponenten

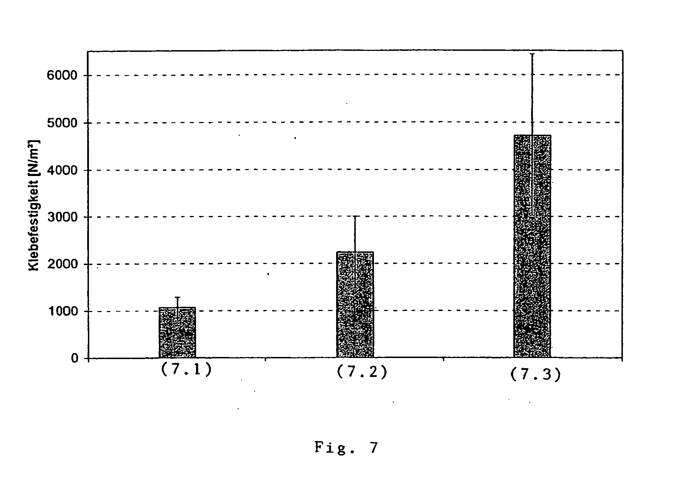

Zur Realisierung praxisüblicher Applikationsbedingungen wurden die sterilisierten Kleberkomponenten in Zweikammerspritzen mit Statikmischer (Firma Mixpac Systems AG, Rotkreuz, Schweiz, Kammerverhältnis 1:1) verabreicht. Für die Untersuchungen wurde das System EOL-NCO (EOUHMDI 1/2 n/n)/DMSO (85/15 w/w, γ-sterilisiert > 25 kGy) als Komponente 1 und Protasan UPCI 213 (medical grade, M_{w} = 270 kDa, 4 %ig in H₂O, wasserdampfsterilisiert 121 °C, 20 min) als Komponente 2 (7.3) mitgeführt. Als Referenzen dienten der auf das Gewebe aufgetragene Fibrinkleber (Tissucol duo S Immuno, Firma Baxter Deutschland GmbH, Unterschleißheim (7.2)) und Gewebeproben ohne Kleberzusatz (7.1). Die Haftfestigkeit wurde aus jeweils 12 Einzelversuchen bestimmt (Figur 7).

### Beispiel 5.4: Durchführung der Klebereaktion für die Hartgewebeklebung bei 37 °C unter Verwendung von Zweikammerspritzen mit sterilisierten Kleberkomponenten

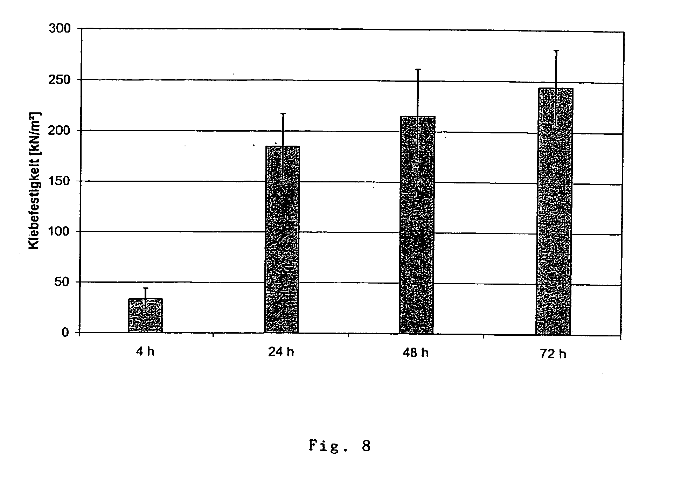

Zur Beurteilung der Haftfestigkeit des synthetisierten Klebersystems auf Hartgewebe wurde die Prüfanordnung modifiziert. Als Hartgewebe-Modellwerkstoff wurde ein offenzelliger Polyurethan-Hartschaum mit Porengrößen von 500 - 1.200 µm verwendet, der mit einer Diamantdrahtsäge (Histo-Saw DDM-P216, Medim) zu quadratischen Prüfkörperplättchen mit einer Geometrie von 15 mm x 15 mm x 3 mm weiterverarbeitet wurde. Die so erhaltenen Einwegprüfkörper wurden anschließend mit 99 %igem Ethanol im Ultraschallbad gereinigt und mittels Cyanacrylat an den quadratischen PMMA-Halterungen für die Aufnahme in der Universalprüfmaschine fixiert. Die sterilisierten Kleberkomponenten wurden ebenfalls mit Hilfe von Zweikammerspritzen mit Statikmischer (Mixpac Systems AG, Rotkreuz, Schweiz, Kammerverhältnis 1:1) auf die fixierten Polyurethanplättchen aufgetragen. Anschließend wurde das Probenpaar leicht zusammengedrückt und im feuchten Milieu verpackt 4 h, 24 h, 48 h und 72 h bei 37 °C gelagert. Die Prüfung der Festigkeit der Klebefuge erfolgte unter einachsiger Zugbeanspruchung an der Universalprüfmaschine Zwick BZ2.5/TN1S (Zwick GmbH & Co. KG, Deutschland; Kraftmeßdose 500 N) ohne die Aufbringung einer definierten Vorkraft mit einer Prüfgeschwindigkeit von 5 mm/min. In der Figur 8 ist der Mittelwert der Meßreihen nach 4 h, 24 h, 48 h und 72 h (jeweils n = 12) von EOL-NCO (EOUHMDI 1/2 n/n)/DMSO (85/15 w/w, γ-sterilisiert > 25 kGy) und Protasan UPCI 213 (medical grade, M_{w} = 270 kDa, 4 %ig in H₂O, wasserdampfsterilisiert 121 °C, 20 min) dargestellt. Der Versuch zeigte, dass die Haftkraft des Klebers nach Applikation zunimmt, was insbesondere für Knochenklebungen und/oder für die Behandlung von Knochendefekten von Vorteil ist.

### Beispiel 5.5: Durchführung der Klebereaktion für die Hartgewebeklebung nach Wassereinlagerung bei 37 °C

Der Einfluß eines Feuchtigkeitseintrags (beispielsweise aufgrund einer Blutung) auf den Polymerisationsvorgang und die Haftfestigkeit des Klebersystems EOL-NCO (EOUHMDI 1/2 n/n)/DMSO (85/15 w/w, γ-sterilisiert > 25 kGy) und Protasan UPCI 213 (medical grade, M_{w} = 270 kDa, 4 %ig in H₂O, wasserdampfsterilisiert 121°C, 20 min) wurde durch Einlagerung präparierter Polurethan-Hartschaumproben in destilliertem Wasser bei 37 °C simuliert. Die Polyurethan-Hartschaumproben wurden analog zu dem vorhergehend beschriebenen Beispiel 5.4 vorbereitet und nach jeweils 10 min, 2 h und 24 h Vorlagerung im feuchten Milieu bei 37 °C weitere 24 h in einem Wasserbad bei 37 °C konditioniert. Die nach Ablauf der jeweiligen Lagerungszeiten gemessenen Klebefestigkeiten belegen, dass die Klebefestigkeit der erfindungsgemäßen Kombination durch Feuchtigkeit im wesentlichen nicht beeinträchtigt wird (vgl. hierzu die in Tabelle 2 wiedergegebenen Werte mit den in Figur 7 abgebildeten Klebefestigkeiten).

**Tabelle 2: Klebefestigkeit von EOL-NCO (EOUHMDI 1/2 n/n)/DMSO (85/15 w/w, γ-sterilisiert) und Protasan UPCI 213 (wasserdampfsterilisiert) auf Polyurethan-Hartschaum bei 37 °C nach 10 min, 2 h und 24 h Vorlagerung im feuchten Milieu und anschließender 24-stündiger Wasserlagerung (jeweils n = 2 Versuche)**

| **Vorlagerung** | **Klebefestigkeit [kN/m²]** |
|---|---|
| 10 min | 196.9 |
| | 222.9 |
| 2 h | 234.7 |
| | 243.6 |
| 24 h | 212.7 |
| | 235.5 |

### Beispiel 5.6: Modifizierung des Klebersystems für die Hartgewebeklebung

Zur Verwendung des Klebersystems als Hartgewebekleber ist es wünschenswert, eine osteoinduktive Wirkung mit Hilfe von Füllstoffen zu erreichen. Hierbei kommen neben Calciumphosphaten, wie beispielsweise Hydroxylapatit und β-Tricalciumphosphat, ebenfalls Carbonate, beispielsweise Calciumcarbonat, oder Oxidmaterialien, beispielsweise Siliziumdioxid, in Frage. Aufgrund des Aufbaus spongiöser Knochenteile ist hierbei eine Korngrößenverteilung der Füllstoffe zwischen 20 nm und 1 µm, vorzugsweise 20 nm und 100 nm, von Vorteil. In diesem Zusammenhang wurde die Eignung von EOL-NCO (EOL HMDI 1/2 n/n)/DMSO (85/15 w/w, γ-sterilisiert) und Protasan UPCI 213 (wasserdampfsterilisiert) als Hartgewebekleber in Verbindung mit Füllstoffen untersucht. Ein Einfluß des Füllstoffs auf die Klebefestigkeit konnte bei einem Füllstoffanteil von 5 % nicht festgestellt werden. Dies wurde anhand von insgesamt 3 Versuchen an einem Polyurethan-Hartschaum als zu verklebendes Substrat verifiziert.

### Beispiel 6:

Bovines Muskelfleisch wurde in rechteckige Stücke mit einer Grundfläche von ca. 2 cm² zugeschnitten. Die zugeschnittenen Stücke wurden auf ca. 37 °C temperiert und in die beiden Klemmbacken einer Zugprüfmaschine der Firma Zwick (Ulm, Deutschland) eingespannt. Die sterile Protasanlösung (vgl. Beispiel 1) und die sterile Lösung des EOL-Isocyanats in DMSO (vgl. Beispiel 2) wurden in einer sterilen Zweikammerspritze vorgelegt und über eine statische Mischspritze vereinigt. Anschließend wurden ca. 200 µl der hieraus entstandenen pastösen Zusammensetzung auf das Fleischstück in der unteren Klemmbacke aufgebracht. Mit einer Vorspannung von ca. 2 Newton wurden die beiden Fleischstücke für ca. 10 Minuten zusammengepresst, bevor bei einer Geschwindigkeit von ca. 10 mm/Min. die Haftkraft des Gewebeklebers bestimmt wurde. In einem Kontrollversuch wurden Fleischstücke mit ca. 200 µl Fibrinkleber (Firma Baxter) verklebt. In einem weiteren Kontrollversuch wurden Fleischstücke ohne Kleber zusammengepresst. Die Ergebnisse aus 12 Messungen sind in der unten stehenden Tabelle 1 aufgeführt. Diese zeigen, dass ein aus einer erfindungsgemäßen Kombination resultierender Kleber eine im Vergleich zum Fibrinkleber deutlich höhere Haftkraft aufweist.

**Tabelle 3**

| | Haftkraft [N/m²] | Standardabweichung |
|---|---|---|
| EOL-Isocyanat-Chitosan | 3705 | 962 |
| Fibrinkleber | 1652 | 366 |
| ohne Kleber | 781 | 296 |

### Beispiel 7:

Analog zu Beispiel 3 wurde schließlich auch die Verklebung von porcinem Lebergewebe untersucht. Die Ergebnisse sind in der nachfolgenden Tabelle 2 aufgelistet.

**Tabelle 4**

| | **Haftkraft [N/m²]** | **Standardabweichung** |
|---|---|---|
| EOL-Isocyanat-Chitosan | 1308 | 664 |
| Fibrinkleber | 553 | 320 |
| ohne Kleber | 118 | 33 |

### Beispiel 8:

In einem weiteren Versuch wurden Muskelfleischstücke analog zu Beispiel 3 mit EOL-NCO-Chitosan einer Zugprüfung unterworfen. Nach Bestimmung der Haftkraft wurden die Fleischstücke nochmals für 10 Minuten zusammengepresst. Dann wurde erneut die aufzubringende Kraft zum Auseinanderziehen der Fleischstücke bestimmt. Die absolute Haftkraft differierte bei insgesamt 12 Versuchen von der anfänglich bestimmten Haftkraft um weniger als 20 %. Mit dem in Beispiel 3 erwähnten Fibrinkleber wurden identische Versuche durchgeführt. Hierbei konnte nach erneutem Zusammenpressen der Fleischstücke keine Haftkraft mehr detektiert werden. Die in diesem Beispiel beschriebene Hafteigenschaft der erfindungsgemäßen Kombination nach Vermischen von deren Komponenten ist besonders beim Fixieren von Knochenbrüchen von großer Bedeutung, bei welchen nach einer anfänglichen Verklebung von zu verbindenden Knochenteilen im weiteren Verlauf der Behandlung Korrekturen durch den Chirurgen erforderlich werden können.

### Beispiel 9: Invitro-Untersuchungen an einem biologischen Gewebe

Es wurde die Druckbeständigkeit einer erfindungsgemäßen Kombination (EOL-Isocyanatlösung in DMSO 80/20 (w/w) und einer 4 %igen Protasanlösung) mit einem zylinderförmigen Biosimulator bestimmt. Der verwendete Biosimulator mit einem druckbeständigen Gehäuse ist 400 mm hoch und hat einen Durchmesser von 150 mm. Der Deckel mit Halterung zum Einspannen von biologischem Gewebe hat ein kreisrundes Loch mit einem Durchmesser von 30 mm. Aufbereitetes, feuchtes Rinderperikard wurde in 60 x 60 mm große Stücke geschnitten und abgetupft. Mit einer Lochstanze wurde zentral ein kreisrunder Defekt mit einem Durchmesser von 10 mm gesetzt und das Perikard in die Halterung des Zylinderdeckels eingebracht. Das zuvor ausgestanzte Stück wurde mit Hilfe der erfindungsgemäßen Kombination auf den gesetzten Defekt geklebt. Nach einer Aushärtezeit von 120 s wurde im Biosimulator mittels Druckluft ein Überdruck erzeugt, wobei der maximale Überdruck bis zum Reißen der Klebestelle detektiert wurde. Bei n = 5 Messungen konnte ein durchschnittlicher Überdruck von 74,3 mbar bestimmt werden. In einem weiteren Versuch wurde ein maximaler Überdruck von 50 mbar eingestellt, der Druck für 1 min gehalten und danach abgelassen. Diese Vorgehensweise wurde 30 x wiederholt, ohne daß die Klebefuge gerissen ist.

Der Versuch zeigt die Druckbeständigkeit und Elastizität der Klebefuge auf biologischem Gewebe. Die erfindungsgemäße Kombination eignet sich daher beispielsweise zum Verschluss von Luftleckagen, wie sie insbesondere in der Lunge nach Resektionen auftreten können.

### Beispiel 10: Toxikologische Untersuchungen

Eine sterile EOL-Isocyanatlösung in DMSO 80/20 (w/w) wurde mit einer 4 %igen Protasanlösung über eine Mixpac-Mischerspitze vermischt und gemäß ISO 10993 (Biological Evaluation of Medical Devices Part 5: Test for Cytotoxicity) einem Zytotoxizitätstest unterworfen. Bei der Auswertung des Tests wurde die EOL-Isocyanat-Protasanmischung ebenso wie die negative Kontrolle mit dem Wert 0 = keine Reaktivität (discrete intracytoplasmic, no cell lysis) bewertet, während die parallel durchgeführte positive Kontrolle mit dem Wert 4 = schwerwiegende Reaktion (nearly complete destruction of the cell layers) zytotoxische Eigenschaften aufwies.

### Beispiel 11: Stillung von Leberblutungen

In einem weiteren Versuch wurde die erfindungsgemäße Kombination zur Stillung von Leberblutungen in Kaninchen eingesetzt. Nach Narkotisierung der Tiere wurde eine Laparatomie durchgeführt, die Leber freigelegt und an jeweils einem Leberlappen eine Teilresektion durchgeführt. Die blutende Leber wurde mit der erfindungsgemäßen Kombination versorgt, wobei deren Komponenten (in diesem Fall eine 4 Gew.-%ige Protasanlösung sowie eine Mischung aus EOL-Isocyanat und DMSO (80/20 w/w) auf der Leberwunde vermischt wurden. Die Blutungen konnten in weniger als 60 Sekunden bei allen Kaninchen gestillt werden.

### Beispiel 12: Stillung von Schürfwunden

Eine sterile EOL-Isocyanatverbindung-DMSO-Mischung 80/20 (w/w) und eine sterile 3 %ige (w/v) Protasanlösung wurden in einem Zweikammerapplikator mit aufgebrachten Spraysystem vorgelegt. Auf der Haut von narkotisierten Ratten wurde mit einem Schmirgelpapier eine 4 x 4 cm große Schürfwunde erzeugt, auf welche die vorstehend genannte Kombination mit drei nacheinander erfolgenden Kolbenhübe aufgetragen wurde. Auf der Wundoberfläche bildete sich ein homogener Film, der die Wunde vollständig abdeckte.

### Beispiel 13: Darmleckagen im Schwein

Im Dickdarm eines Schweins wurden nach dessen Narkotisierung mit einer spitzen Kanüle mehrere Punktionen gesetzt. Eine sterile EOL-Isocyanatlösung in DMSO 80/20 (w/w) wurde mit einer 4 %igen Protasanlösung über eine Mixpac-Mischerspitze vermischt und auf die Punktionsstellen aufgetragen. Die milchig-weiße Trübung erleichtert dem Chirurgen die zielgenaue Applikation des Klebers auf dem Gewebe. Parallel wurden Punktionsstellen auch mit einer reinen zähflüssigen EOL-Isocyanatlösung bzw. mit einer reinen 4 %igen Chitosanlösung versorgt. Nach einer Aushärtungszeit von 180 s wurde die Luftdichtigkeit der Punktion durch Zusammendrücken des Darms überprüft. Die mit der erfindungsgemäßen Kombination versorgten Punktionsstellen erwiesen sich dabei als dicht bzw. abgedichtet, während die mit einer reinen EOL-Isocyanatlösung bzw. reinen 4 %igen Chitosanlösung behandelten Punktionsstellen undicht waren, was anhand von entweichenden Luftblasen festgestellt werden konnte.

### Beispiel 14: Stichkanalblutungen

Nach Narkotisierung der Schweine (n = 3) und Öffnung des Abdomens wurde die infrarenale Aorta frei präpariert. Ein ca. 3 cm langer Gefäßabschnitt wurde explantiert und durch eine PTFE-Prothese im Rahmen einer "end to end-Anastomose" substituiert. Beim Öffnen der Gefäßklemmen waren an beiden Anastomosen Stichkanalblutungen zu sehen, die nach erneutem Anlegen der Gefäßklemmen mit einer erfindungsgemäßen Kombination (EOL-Isocyanatlösung in DMSO 80/20 (w/w) und 4 %ige Protasanlösung) versorgt wurden. Die milchig-weiße Trübung der erhaltenen Mischung erleichterte dabei dem Chirurgen die zielgenaue Applikation des Klebers auf dem Gewebe. Die Gefäßklemmen wurden 30 s nach Applikation der erfindungsgemäßen Kombination erneut geöffnet. Es traten keine Stichkanalblutungen mehr auf.

Die vorstehend beschriebenen Tierversuche zeigen die Funktionalität der erfindungsgemäßen Kombination zum Verschluss von Flüssigkeits- und Luftleckagen. Die bei Vermischen der Komponenten a) und b) der Kombination auftretende milchig-weiße Trübung erleichtert dem Chirurgen die Unterscheidbarkeit zwischen Kombination und zu versorgendem Gewebe, wodurch sich die Kombination mit besonderem Vorteil zielgenau applizieren läßt.

## Patentansprüche

1. Kombination, insbesondere zum Verkleben und/oder Fixieren von biologischen und/oder synthetischen Geweben, umfassend die Komponenten
a) ein stickstofffunktionalisiertes Polysaccharid
b) ein endständig funktionalisiertes Oligolacton, **dadurch gekennzeichnet, dass** das Polysaccharid und das Oligolacton getrennt voneinander vorliegen und das Oligolacton endständige Isocyanatgruppen aufweist.

2. Kombination nach Anspruch 1, **dadurch gekennzeichnet, dass** das Polysaccharid ein natürlich vorkommendes Polysaccharid oder von einem solchen abgeleitet ist.

3. Kombination nach Anspruch 1 oder 2, **dadurch gekennzeichnet, dass** das Polysaccharid ein Aminogruppen, insbesondere primäre Aminogruppen, tragendes Polysaccharid ist.

4. Kombination nach einem der vorhergehenden Ansprüche, **dadurch gekennzeichnet, dass** der Anteil an Monosaccharideinheiten im Polysaccharid, die eine Aminogruppe tragen, mindestens 30 %, vorzugsweise 80 %, beträgt.

5. Kombination nach einem der vorhergehenden Ansprüche, **dadurch gekennzeichnet, dass** das Polysaccharid ein zumindest teilweise deacetyliertes Glykosaminoglykan (Mucopolysaccharid) ist.

6. Kombination nach einem der vorhergehenden Ansprüche, **dadurch gekennzeichnet, dass** das Polysaccharid einen Deacetylierungsgrad zwischen 50 und 98%, insbesondere zwischen 60 und 95%, vorzugsweise zwischen 80 und 90%, aufweist.

7. Kombination nach einem der vorhergehenden Ansprüche, **dadurch gekennzeichnet, dass** das Polysaccharid Chitosan oder ein Derivat davon ist.

8. Kombination nach einem der vorhergehenden Ansprüche, **dadurch gekennzeichnet, dass** das Oligolacton aus einem mehrwertigen Alkohol und Hydroxycarbonsäuren, insbesondere Glykol- und/oder Milchsäure, gebildet ist.

9. Kombination nach einem der vorhergehenden Ansprüche, **dadurch gekennzeichnet, dass** der Anteil an Polysaccharid in der Kombination 1 bis 80 Gew.-%, insbesondere 5 bis 70 Gew.-%, beträgt.

10. Kombination nach einem der vorhergehenden Ansprüche, **dadurch gekennzeichnet, dass** der Anteil an Oligolacton in der Kombination 20 bis 99 Gew.-%, insbesondere 30 bis 95 Gew.-%, beträgt.

11. Kombination nach einem der vorhergehenden Ansprüche, **dadurch gekennzeichnet, dass** das Polysaccharid und/oder das Oligolacton in einem oder mehreren Lösungsmitteln dispergiert, vorzugsweise gelöst, vorliegen.

12. Kombination nach einem der vorhergehenden Ansprüche, **dadurch gekennzeichnet, dass** das Polysaccharid in Form einer wässrigen Lösung, insbesondere mit einem Polysaccharidanteil von 2 bis 5 Gew.-%, vorzugsweise 3 bis 4 Gew.-%, bezogen auf das Gesamtgewicht der Lösung, vorliegt.

13. Kombination nach einem der vorhergehenden Ansprüche, **dadurch gekennzeichnet, dass** das Oligolacton in Form einer DMSO-Lösung, insbesondere mit einem Oligolactonanteil zwischen 50 und 99 Gew.-%, bezogen auf das Gesamtgewicht der Lösung, vorliegt.

14. Kombination nach einem der vorhergehenden Ansprüche, **dadurch gekennzeichnet, dass** das Polysaccharid und das Oligolacton getrennt voneinander in den Kammern einer Mehrkomponentenspritze vorliegen.

15. Kombination nach einem der vorhergehenden Ansprüche, **dadurch gekennzeichnet, dass** eine Reaktion, vorzugsweise Vernetzung, der Komponenten nach ihrer Vermischung auf einem zu klebenden Substrat innerhalb eines Zeitraumes von < 100 Sekunden, insbesondere < 80 Sekunden, vorzugsweise < 60 Sekunden, initiiert wird.

16. Kombination nach einem der vorhergehenden Ansprüche, **dadurch gekennzeichnet, dass** die Kombination Zusatzstoffe, insbesondere Kollagen, Gelatine und/oder Albumin, aufweist.

17. Kombination nach einem der vorhergehenden Ansprüche, **dadurch gekennzeichnet, dass** das Polysaccharid und/oder das Oligolacton in sterilisierter Form vorliegen.

18. Kombination nach einem der vorherigen Ansprüche, **dadurch gekennzeichnet, dass** es sich bei der Kombination um eine Klebstoffzusammensetzung handelt.

19. Verbindung, **dadurch gekennzeichnet, dass** die Komponenten der Kombination nach einem der vorhergehenden Ansprüche vermischt sind und das Polysaccharid zumindest teilweise mit dem Oligolacton vernetzt ist.

20. Medizintechnisches Produkt, insbesondere zum Verkleben und/ oder Fixieren von biologischen und/oder synthetischen Geweben, umfassend eine Kombination nach einem der vorhergehenden Ansprüche 1 bis 18.

21. Kit, umfassend mindestens zwei getrennte Behältnisse, wobei das eine Behältnis ein stickstofffunktionalisiertes Polysaccharid und das andere Behältnis ein endständig funktionalisiertes Oligolacton enthält, **dadurch gekennzeichnet, dass** das Oligolacton endständige Isocyanatgruppen aufweist.

22. Kit nach Anspruch 21, weiter **gekennzeichnet durch** ein stickstofffunktionalisiertes Polysaccharid und/oder endständig funktionalisiertes Oligolacton nach einem der Ansprüche 2 bis 19.

23. In-vitro-Verfahren zum Kleben, **dadurch gekennzeichnet, dass** die Komponenten der Kombination nach einem der Ansprüche 1 bis 18 miteinander und mit mindestens einem zu klebenden Substrat, das reaktive funktionelle Gruppen aufweist, in Kontakt gebracht werden.

24. Verwendung einer Kombination nach einem der Ansprüche 1 bis 18 zur Herstellung eines medizintechnischen Produktes.

## Claims

1. A combination, in particular for the adhesive bonding and/or fixing of biological and/or synthetic tissues, comprising the components
a) a nitrogen-functionalized polysaccharide,
b) a terminally functionalized oligolactone, **characterized in that** the polysaccharide and the oligolactone are present separately from one another, and **in that** the oligolactone has terminal isocyanate groups.

2. The combination as claimed in claim 1, **characterized in that** the polysaccharide is a naturally occurring polysaccharide or derived from one such.

3. The combination as claimed in claim 1 or 2, **characterized in that** the polysaccharide is a polysaccharide bearing amino groups, in particular primary amino groups.

4. The combination as claimed in one of the previous claims, **characterized in that** the content of monosaccharide units in the polysaccharide which bear an amino group is at least 30 %, preferably 80 %.

5. The combination as claimed in one of the previous claims, **characterized in that** the polysaccharide is an at least partly deacetylated glycosaminoglycan (mucopolysaccharide).

6. The combination as claimed in one of the previous claims, **characterized in that** the polysaccharide has a deacetylation level between 50 and 98 %, in particular between 60 and 95 %, preferably between 80 and 90 %.

7. The combination as claimed in one of the previous claims, **characterized in that** the polysaccharide is chitosan or a derivative thereof.

8. The combination as claimed in one of the previous claims, **characterized in that** the oligolactone is formed from a polyhydric alcohol and hydroxy-carboxylic acids, in particular glycolic and/or lactic acid.

9. The combination as claimed in one of the previous claims, **characterized in that** the content of poly-saccharide in the combination is 1 to 80 wt. %, in particular 5 to 70 wt. %.

10. The combination as claimed in one of the previous claims, **characterized in that** the content of oligolactone in the combination is 20 to 99 wt. %, in particular 30 to 95 wt. %.

11. The combination as claimed in one of the previous claims, **characterized in that** the polysaccharide and/or the oligolactone are present dispersed, preferably dissolved, in one or more solvents.

12. The combination as claimed in one of the previous claims, **characterized in that** the polysaccharide is present in the form of an aqueous solution, in particular with a polysaccharide content of 2 to 5 wt. %, preferably 3 to 4 wt. %, based on the total weight of the solution.

13. The combination as claimed in one of the previous claims, **characterized in that** the oligolactone is present in the form of a DMSO solution, in particular with an oligolactone content between 50 and 99 wt. %, based on the total weight of the solution.

14. The combination as claimed in one of the previous claims, **characterized in that** the polysaccharide and the oligolactone are present separately from one another in the chambers of a multicomponent syringe.

15. The combination as claimed in one of the previous claims, **characterized in that** a reaction, preferably crosslinking, of the components after mixing thereof on a substrate to be adhesively bonded is initiated within a period of < 100 seconds, in particular < 80 seconds, preferably < 60 seconds.

16. The combination as claimed in one of the previous claims, **characterized in that** the combination contains additives, in particular collagen, gelatin and/or albumin.

17. The combination as claimed in one of the previous claims, **characterized in that** the polysaccharide and/or the oligolactone are present in sterilized form.

18. The combination as claimed in one of the previous claims, **characterized in that** the combination is an adhesive composition.

19. A compound **characterized in that** the components of the combination as claimed in one of the previous claims are mixed and the polysaccharide is at least partly crosslinked with the oligolactone.

20. A medico-technical product, in particular for the adhesive bonding and/or fixing of biological and/or synthetic tissues, comprising a combination as claimed in one of the previous claims 1 to 18.

21. A kit, comprising at least two separate containers, wherein one container contains a nitrogen-functionalized polysaccharide and the other container contains a terminally functionalized oligolactone, **characterized in that** the oligolactone has terminal isocyanate groups.

22. The kit as claimed in claim 21, further **characterized by** a nitrogen-functionalized polysaccharide and/or terminally functionalized oligolactone as claimed in one of claims 2 to 19.

23. An in vitro process for adhesive bonding, **characterized in that** the components of the combination as claimed in one of claims 1 to 18 are brought into contact with one another and with at least one substrate to be bonded, which contains reactive functional groups.

24. Use of a combination as claimed in one of claims 1 to 18 for the production of a medico-technical product.

## Revendications

1. Combinaison, en particulier pour le collage et/ou la fixation de tissus biologiques et/ou synthétiques, comprenant les composants
a) un polysaccharide fonctionnalisé à l'azote,
b) une oligolactone fonctionnalisée en position terminale, **caractérisée en ce que** le polysaccharide et l'oligolactone sont présents séparés l'un de l'autre, et l'oligolactone comprend des groupes isocyanate terminaux.

2. Combinaison selon la revendication 1, **caractérisée en ce que** le polysaccharide est un polysaccharide naturel ou dérive d'un tel polysaccharide.

3. Combinaison selon la revendication 1 ou 2, **caractérisée en ce que** le polysaccharide est un polysaccharide portant des groupes amino, en particulier des groupes amino primaires.

4. Combinaison selon l'une des revendications précédentes, **caractérisée en ce que** la proportion, dans le polysaccharide, de motifs monosaccharidiques portant un groupe amino, est d'au moins 30 %, de préférence de 80 %.

5. Combinaison selon l'une des revendications précédentes, **caractérisée en ce que** le polysaccharide est un glycosaminoglycane au moins partiellement désacétylé (mucopolysaccharide).

6. Combinaison selon l'une des revendications précédentes, **caractérisée en ce que** le polysaccharide présente un degré de désacétylation compris entre 50 et 98 %, en particulier entre 60 et 95 %, de préférence entre 80 et 90 %.

7. Combinaison selon l'une des revendications précédentes, **caractérisée en ce que** le polysaccharide est le chitosane ou un dérivé de ce dernier.

8. Combinaison selon l'une des revendications précédentes, **caractérisée en ce que** l'oligolactone est formée à partir d'un polyalcool et d'acides hydroxycarboxyliques, en particulier l'acide glycolique et/ou l'acide lactique.

9. Combinaison selon l'une des revendications précédentes, **caractérisée en ce que** la proportion du polysaccharide dans la combinaison est de 1 à 80 % en poids, en particulier de 5 à 70 % en poids.

10. Combinaison selon l'une des revendications précédentes, **caractérisée en ce que** la proportion de l'oligolactone dans la combinaison est de 20 à 99 % en poids, en particulier de 30 à 95 % en poids.

11. Combinaison selon l'une des revendications précédentes, **caractérisée en ce que** le polysaccharide et/ou l'oligolactone se présentent sous forme dispersée, de préférence dissoute, dans un ou plusieurs solvants.

12. Combinaison selon l'une des revendications précédentes, **caractérisée en ce que** le polysaccharide se présente sous forme d'une solution aqueuse, la proportion du polysaccharide, rapportée au poids total de la solution, étant en particulier de 2 à 5 % en poids, de préférence de 3 à 4 % en poids.

13. Combinaison selon l'une des revendications précédentes, **caractérisée en ce que** l'oligolactone se présente sous forme d'une solution dans le DMSO, la proportion de l'oligolactone, rapportée au poids total de la solution, étant en particulier de 50 à 99 % en poids.

14. Combinaison selon l'une des revendications précédentes, **caractérisée en ce que** le polysaccharide et l'oligolactone sont présents séparés l'un de l'autre dans les compartiments d'une seringue multicompartiments.

15. Combinaison selon l'une des revendications précédentes, **caractérisée en ce qu'**on amorce une réaction, de préférence une réticulation, des composants après leur mélange sur un substrat à coller, sur un laps de temps < 100 secondes, en particulier < 80 secondes, de préférence < 60 secondes.

16. Combinaison selon l'une des revendications précédentes, **caractérisée en ce que** la composition comprend des additifs, en particulier du collagène, de la gélatine et/ou de l'albumine.

17. Combinaison selon l'une des revendications précédentes, **caractérisée en ce que** le polysaccharide et/ou l'oligolactone se présentent sous forme stérilisée.

18. Combinaison selon l'une des revendications précédentes, **caractérisée en ce que**, pour ce qui concerne la combinaison, il s'agit d'une composition adhésive.

19. Composé, **caractérisé en ce que** les composants de la combinaison sont mélangés selon l'une des revendications précédentes, et le polysaccharide est au moins partiellement réticulé avec l'oligolactone.

20. Produit de technique médicale, en particulier pour le collage et/ou la fixation de tissus biologiques et/ou synthétiques, comprenant une combinaison selon l'une des revendications précédentes 1 à 18.

21. Trousse comprenant au moins deux récipients distincts, l'un des récipients contenant un polysaccharide fonctionnalisé à l'azote et l'autre récipient contenant une oligolactone fonctionnalisée en position terminale, **caractérisée en ce que** l'oligolactone comprend des groupes isocyanate terminaux.

22. Trousse selon la revendication 21, **caractérisée en outre par** un polysaccharide fonctionnalisé à l'azote et/ou une oligolactone fonctionnalisée en position terminale selon l'une des revendications 2 à 19.

23. Procédé *in vitro* de collage, **caractérisé en ce que** les composants de la combinaison selon l'une des revendications 1 à 18 sont mis en contact les uns avec les autres et avec au moins un substrat à coller, qui comprend des groupes fonctionnels réactifs.

24. Utilisation d'une combinaison selon l'une des revendications 1 à 18 pour fabriquer un produit de technique médicale.
